# EUROPEAN PATENT APPLICATION

(11) **EP 4 850 620 A1**
(43) Date of publication of application: **09.12.2026**
(21) Application number: 25180505.7
(22) Date of filing: 03.06.2025
(51) Int. Cl.: A61B 17/34, H01F 13/00, A61B 17/00, A61B 34/20, A61B 90/00

(54) **METHOD AND APPARATUS FOR PROCESSING AN ELONGATED BODY**

(71) Applicant: eZono AG, 07743 Jena (DE)
(72) Inventor: The designation of the inventor has not yet been filed
(74) Representative: J A Kemp LLP

(57) **Abstract**

Methods and apparatus for processing an elongate body are disclosed. A method comprises: moving an elongated body into and out of a first processing region associated with a first magnetizer. The first magnetizer generates a first magnetic field pattern in the first processing region. The first magnetic field pattern comprises a magnetizing zone in which a magnetic field direction is predominantly aligned with a longitudinal axis of the elongated body and is perpendicular to a direction of movement of the elongated body while the elongated body is moving into the first processing region. The first magnetic field pattern magnetizes the elongated body with a magnetization pattern defined by the first magnetic field pattern in the first processing region. The magnetization pattern is retained by permanent magnetism in the elongated body after the elongated body has left the first processing region.

## Description

The present disclosure relates to methods and apparatus for processing an elongated body. The disclosure is particularly applicable to processing elongated bodies that are to be used as medical instruments.

Many medical procedures use elongated bodies as medical instruments. Such procedures may involve insertion of an elongated body into a subject and/or manipulation of the elongated body within the subject. Examples of such elongated bodies include needles, cannulas, catheters and stylets. Examples of such medical procedures include minimally-invasive surgical procedures, local anaesthesia, detection of bioelectrical signals, electrical stimulation for diagnosis or treatment, vascular access, fine needle aspiration, radio frequency ablation, biopsy and musculoskeletal injections. The procedures may require accurate guiding of the elongated body to a desired position in the subject's body. However, it can be challenging to monitor the position and/or orientation of the elongated body during movement inside the patient's body.

Ultrasound imaging has been used to guide needle and catheterisation procedures. However, for various technical and handling reasons, it is difficult to track needles in ultrasound images. Practitioners may also rely on their skill and experience to judge positioning of medical instruments based on feel, but this requires extensive training and is prone to human error.

Magnetic tracking of medical instruments has been proposed as an alternative approach. A magnetometric detector may, for example, be attached to a freehand ultrasound probe and used to detect the position and/or orientation of a magnetised tissue-penetrating tool (an example of an elongated body). According to this approach, a freehand ultrasound probe may comprise an array of magnetometric sensors that detect the magnetic field from the magnetised tissue-penetrating tool. A base station may then calculate a relative position and orientation of the tissue-penetrating medical tool relative to the ultrasound probe based on measurements performed by the sensors. The base station can supply this calculated position and orientation to the ultrasound imaging system so that the tissue-penetrating medical tool can be displayed on the ultrasound image of the subject's anatomy.

The system is advantageous in that it allows the operator to see both the ultrasound imaged anatomy and the magnetically detected tissue-penetrating medical tool on the same image. This enables greater accuracy in the procedure. Further, the attachment of a magnetometric detector to the ultrasound probe does not alter the feel of the ultrasound probe significantly, and it remains, therefore, familiar to the practitioner. Similarly, the magnetization of the tissue-penetrating medical tool does not alter its mechanical characteristics, which again preserves familiarity for the clinician and enables best use to be made of previous experience. Because the ultrasound probe can be manipulated freely, the ease-of-use of the freehand ultrasound system is also preserved.

It can be challenging to provide magnetised tissue-penetrating tools that reliably have an optimal magnetization at the point of use. Magnetization as a single dipole may not be adequate for tissue-penetrating tools that are relatively long. Magnetization with multiple dipoles may be complex to implement, particularly at the point of use.

US9257220B2 discloses a method of magnetization involving movement of a tissue-penetrating medical tool through a space to magnetize it. The disclosed magnetization method involves a receptacle adapted to permit movement of the tissue-penetrating medical tool relative to the receptacle in a direction parallel to a longitudinal axis of the tissue-penetrating medical tool. The processing requires a reciprocating movement of the medical tool into and out of the space in the receptacle, which can be complex to implement reliably and may undesirably limit throughput in situations where large numbers of medical tools need to be processed.

US 2023/0101371 A1 discloses a magnetic signature imprinting system for imprinting a magnetic signature into an elongate medical device. The medical device is fed axially through coils of one or more electromagnets. The processing requires relatively complex control of movement of the medical device and is difficult to scale to high throughout scenarios involving processing of many medical devices.

US 2023/0102059 A1 discloses a magnetic signature identification system that uses magnetometers to detect magnetic characteristics defined by magnetic elements such as dipoles coupled with a target medical device. The disclosed system determines an identity of a target medical device by a comparison of a detected magnetic signature of the target medical device with multiple magnetic signatures stored in a memory and associated with different medical devices.

It is an object of the invention to provide methods and apparatus supporting the provision of improved medical instruments for use in surgical operations that involve magnetic detection of the position and/or orientation of the medical instruments.

According to an aspect of the disclosure, there is provided a method of processing an elongated body, comprising: moving an elongated body into and out of a first processing region associated with a first magnetizer, wherein: the first magnetizer generates a first magnetic field pattern in the first processing region, the first magnetic field pattern comprising a magnetizing zone in which a magnetic field direction is predominantly aligned with a longitudinal axis of the elongated body and is perpendicular to a direction of movement of the elongated body while the elongated body is moving into the first processing region; and the first magnetic field pattern magnetizes the elongated body with a magnetization pattern defined by the first magnetic field pattern in the first processing region, the magnetization pattern being retained by permanent magnetism in the elongated body after the elongated body has left the first processing region.

Thus, a method is provided that allows elongated bodies to be magnetized efficiently and reliably. The method is particularly applicable to implementation during bulk manufacture of elongated bodies. The method may be provided, for example, as part of a production line of magnetized elongated bodies. Applying a magnetic field substantially perpendicularly to the direction of movement of the elongated bodies facilitates provision of a conveyance arrangement for providing the relative movement, as well as improving compatibility with existing conveyance arrangements for bulk processing of elongated bodies for medical use. The method also facilitates application of a range of different magnetization patterns if desired.

Optionally, the first magnetizer generates the first magnetic field pattern at least partially using one or more permanent magnets. Using permanent magnets to provide the desired magnetic field pattern avoids the need for complex power supplies and controllers that may be associated with alternative approaches that use driven electrical currents to generate magnetic fields, as well as providing robust and reliable performance.

Optionally, the one or more magnets are supported by a mounting arrangement configured to allow the one or more magnets to be moved relative to a conveyance arrangement, the conveyance arrangement being configured to convey the elongated body through the first processing region of the first magnetizer. Configuring the permanent magnets to be movable in this way provides flexibility for varying the magnetization pattern applied to the elongated body without introducing excessive system complexity.

Optionally, the mounting arrangement is configured to allow movement of one or more of the magnets in a direction parallel to the longitudinal axis. Moving a magnet parallel to the longitudinal axis provides a mechanically simple and robust way of varying the position of the magnetic field applied to the elongated body by the magnet, enabling the magnet to magnetize different portions of the elongated body, or to be moved away from the elongated body to apply magnetization to only a portion of the elongated body.

Optionally, the mounting arrangement is configured to allow movement of one or more of the magnets in a direction perpendicular to the longitudinal axis. Moving a magnet perpendicular to the longitudinal axis provides a mechanically simple and robust way of varying a separation between the magnet and the elongated body, thereby varying the strength of magnetic field applied to the elongated body by the magnet. The perpendicular movement may allow magnets to be effectively "switched on" and "switched off" with respect to their effect on the elongated body.

Optionally, the one or more magnets comprises one or more pairs of magnets. The magnets of each pair of magnets may be positioned on opposite sides of the first processing region in a direction perpendicular to the movement of the elongated body into and out of (e.g., through) the first processing region. Positioning pairs of magnets in this way facilitates provision of a relatively large volume of magnetized material close to the elongated body, thereby facilitating effective magnetization of the elongated element without requiring excessively powerful and/or large permanent magnets. The use of pairs of magnets may also facilitate application of a magnetic field that is mirror symmetric about the longitudinal axis of the elongated body, at least in a plane passing through the magnets of the pair.

Optionally, the plurality of magnets are supported by a mounting arrangement containing one or more field-guiding bodies, each field-guiding body being formed from a material having a relative magnetic permeability greater than one, optionally greater than 500, optionally greater than 5000, optionally greater than 10000. The field-guiding bodies may promote efficient magnetization of the elongated body by concentrating magnetic fields associated with the magnets in zones of interest.

Optionally, wherein the one or more field-guiding bodies are configured to inhibit spreading of a magnetic field from the one or more permanent magnets outside of the magnetizing zone. Inhibiting such spreading increases the strength of the magnetic field in the magnetizing zone as well as reducing or avoiding application of a magnetic field having significant components oriented perpendicularly to the longitudinal axis of the elongated body while the elongated body is approaching the magnetizing zone or moving away from the magnetizing zone. The quality of the magnetization applied to the elongated body may thereby be improved.

Optionally, the first magnetizer generates the first magnetic field at least partially using an electromagnet. Using an electromagnet provides increased flexibility for controlling the magnetic field in the first processing region, allowing for example for the magnetic field to be turned on and off quickly without requiring mechanical movement of any elements.

Optionally, the method comprises controlling the electromagnet to: generate the first magnetic field pattern when the elongated body is in the first processing region; and not generate the first magnetic field pattern when the elongated body is outside of the first processing region, optionally including when the elongated body is approaching the first processing region before moving into the first processing region and/or when the elongated body is moving away from the first processing region after moving out of the first processing region. Controlling the electromagnet to only generate the magnetic field when the elongated body is positioned in the first processing region may promote application of the magnetic field in a desired orientation, such as parallel to the longitudinal axis and/or reduce or avoid disruption of the magnetization applied to the elongated body that may otherwise arise due to stray fields acting on the elongated element while it is approaching the first processing region or moving away from the first processing region.

Optionally, the elongated body is moved by a conveyance arrangement configured to convey a plurality of the elongated bodies in sequence into and out of (e.g., through) the first processing region of the first magnetizer . Use of such a conveyance mechanism allows the method to efficiently process a large number of elongated bodies.

Optionally, the method comprises moving the elongated body into and out of (e.g., through) one or more second processing regions of respective one or more second magnetizers, each second magnetizer being configured to generate a second magnetic field pattern in the second processing region that is different from the first magnetic field pattern in the first processing region. Passing the elongated body through multiple different magnetic field patterns facilitates provision of complex patterns of magnetization in the elongated body, including avoiding or reducing the need for individual magnetizers to have a complex structure, reducing or avoiding the need for individual permanent magnets to be excessively small, and/or facilitating the provision of magnetic field strengths in the processing region that are sufficiently large to ensure reliable magnetization of the elongated body.

Optionally, the method further comprises measuring the retained magnetization pattern after the elongated body has left the first processing region (and/or after the elongated body has left any second processing region) and evaluating a quality of the retained magnetization pattern based on the measurement. Actively checking the magnetization pattern allows malfunctions to be detected early and/or ensures that only correctly magnetized elongated bodies are output for use. The measurements may also be used as feedback to optimise adjustable parameters of the method, such as the positioning of magnets and/or elongated bodies and/or the speed at which elongated bodies are conveyed through the processing regions.

According to an aspect of the disclosure, there is provided an apparatus for processing an elongated body, comprising: a first magnetizer configured to generate a first magnetic field pattern in a first processing region; and a conveyance arrangement configured to move an elongated body into and out of the first processing region, wherein: the first magnetic field pattern comprises a magnetizing zone in which a magnetic field direction is aligned with a longitudinal axis of the elongated body and is perpendicular to a direction of movement of the elongated body while the elongated body is moving into the first processing region; and the first magnetic field pattern is configured to magnetize the elongated body with a magnetization pattern defined by the first magnetic field pattern in the first processing region, the magnetization pattern being retained by permanent magnetism in the elongated body after the elongated body has left the first processing region.

According to an aspect of the disclosure, there is provided a method of assessing a magnetization pattern in an elongate body, comprising: holding the elongate body at a predetermined location and orientation relative to one or more magnetic field sensors; using the one or more magnetic field sensors to measure a magnetic characteristic of the elongate body while the elongate body is held at the predetermined location and orientation; comparing the measured magnetic characteristic with an expected magnetic characteristic associated with a target magnetization pattern; and determining a quality of a magnetization pattern of the elongate body based on the comparison.

According to an aspect of the disclosure, there is provided an apparatus for assessing a magnetization pattern in an elongate body, comprising: one or more magnetic field sensors; a holder configured to hold an elongate body at a predetermined location and orientation relative to the one or more magnetic field sensors; and a data processing system configured to: cause the one or more magnetic field sensors to measure a magnetic characteristic of the elongate body; compare the measured magnetic characteristic with an expected magnetic characteristic associated with a target magnetization pattern; and determine a quality of a magnetization pattern of the elongate body based on the comparison.

Embodiments of the disclosure will be further described by way of example only with reference to the accompanying drawings.
Figure 1 is a schematic side view of an apparatus for implementing methods of processing an elongated body according to the present disclosure.
Figure 2 is a schematic end sectional view of a magnetizer of the apparatus of Figure 1 with an elongated body positioned within a first processing region of the magnetizer.
Figure 3 is a schematic end sectional view of an example elongated body after processing by the magnetizer of Figure 2.
Figure 4 is a schematic view looking outwards at a portion of a magnetizer from a location of an elongated body in a processing region of the magnetizer.
Figure 5 is a schematic view looking at the magnetizer of Figure 4 along a direction of movement of an elongated body.
Figure 6 is a schematic perspective view of an apparatus for implementing methods of processing an elongated body according to the present disclosure.
Figures 7-9 are schematic views along a conveyance direction of an elongated body in a processing region of a magnetizer, the views showing relative positions of the elongated body and magnets of the magnetizer for different positions of a support structure relative to the conveyance arrangement and elongated body. Figure 7 depicts a case where the support structure is at an intermediate height. Figure 8 depicts a case where the support structure is raised relative to the case of Figure 7. Figure 9 depicts a case where the support structure is lowered relative to the case of Figure 7.

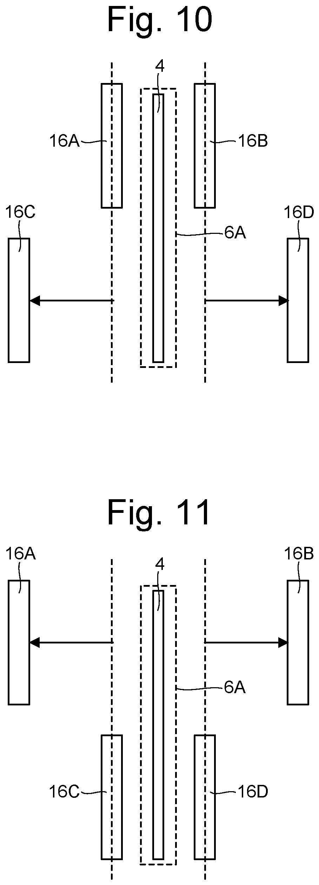

Figures 10-11 are schematic views along a conveyance direction of an elongated body in a processing region of a magnetizer. The views show relative positions of the elongated body and permanent magnets of the magnetizer. Figure 10 depicts a configuration in which a lowermost pair of magnets have been moved horizontally away from the elongated body while an uppermost pair of magnets are positioned closer to the elongated body. Figure 11 depicts an alternative configuration in which the uppermost pair of magnets have been moved horizontally away from the elongated body and the lowermost pair of magnets are positioned closer to the elongated body.
Figure 12 is a graph showing simulated magnetic field lines in the Y-Z plane from an example configuration of two columns of permanent magnets. The columns are positioned at y = -6 and y = +6 and arranged S-N, N-S, S-N, N-S from top to bottom. The elongated body is aligned along the Z axis and moves along the X axis (into the plane of the page). This arrangement of magnets creates a strong magnetic field at the elongated body that is oriented parallel to the magnets and to the longitudinal axis of the elongated body.
Figure 13 is a graph showing simulated magnetic field lines in the X-Z plane from the same configuration of magnets as Figure 12. The elongated body moves from x = -10 to x = 10. When x = 0 the elongated body is in between the two columns of magnets. The magnets have a width extended from x = -5 to x = 5, and a height extending from z = 0 to z = 120mm. As can be seen, the unconstrained magnetic field has very strong horizontal components in the exit trajectory of the needle.

References are made below to room temperature in relation to describing magnetic properties of materials, which typically vary as a function of temperature. Room temperature is understood in this context to be 298K, although in practice the magnetic properties in question will be substantially the same through a range of temperatures at which medical instruments may conceivably be used, such as in a range of about 30K above and below 298K. The magnetic properties in question will also typically be substantially the same through a broader range of temperatures that goes beyond those at which medical instruments may conceivably be used during an operation, including temperatures that may be used for sterilization prior to an operation.

The disclosure provides a method of processing an elongated body 4. The processed elongated body 4 may form all or part of a medical instrument. The method may thus form part of a method of manufacturing a medical instrument.

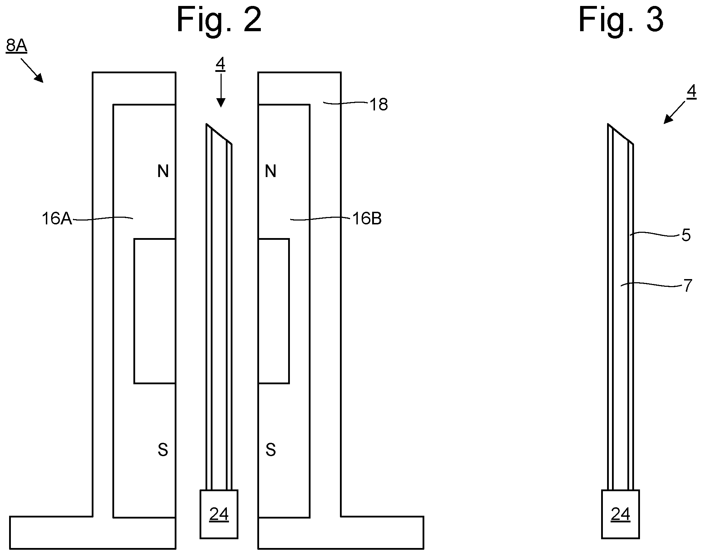

The method may be applied to a wide range of elongated bodies 4 suitable for use as medical instruments. The method is particularly applicable to tissue-penetrating tools. The elongated body 4 may comprise a portion or all of one or more of the following: a needle, a cannula, a stylet, a guidewire, and a cable. The elongated body 4 may therefore take various forms, including one or more of the following: a solid elongated body (cylindrical or otherwise); a hollow elongated body (cylindrical or otherwise); a composite structure comprising a core wire and a coiled wire around the core wire (e.g., to form a guidewire); and a solid or elongated body (cylindrical or otherwise) with one or more longitudinal recesses (which may be referred to as channels) running along a portion or all of the length of the body. In the examples discussed below, as depicted particularly in Figure 3, the elongated body 4 is a hollow cannula comprising a cylindrical body 5 defining an inner lumen 7, but the apparatus and methods described could equally be used with any other suitable type of elongated body 4 for a medical instrument, including any of those listed above.

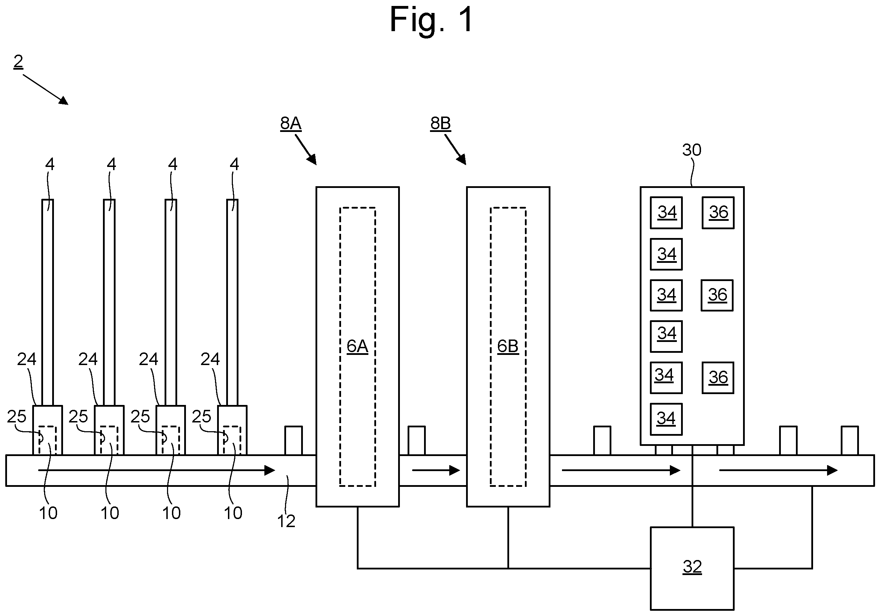

Figure 1 depicts an example apparatus 2 for performing methods of the disclosure. The apparatus 2 comprises a first magnetizer 8A. Figures 2, 4, 5 and 6 depict example configurations for the first magnetizer 8A. Figure 3 depicts an elongated body 4 after processing by the apparatus 2. The first magnetizer 8A is associated with a first processing region 6A. The method comprises moving an elongated body 4 into and out of (e.g., through) the first processing region 6A. The elongated body 4 may be moved continually (e.g., without stopping) through the first processing region 6A. Alternatively, the elongated body 4 may be moved into the first processing region 6A, stopped temporarily in the first processing region 6A, and then moved out of the first processing region 6A. In the example of Figure 1, the apparatus 2 is configured to apply the method to each of a plurality of elongated bodies 4 in sequence. The elongated bodies 4 are moved in sequence, one after the other from left to right in the orientation of Figure 1, into and out of (e.g., through) the first processing region 6A.

The first magnetizer 8A generates a first magnetic field pattern in the first processing region 6A. The first magnetic field pattern comprises one or more magnetizing zones. In each magnetizing zone, a magnetic field direction is predominantly aligned with a longitudinal axis of the elongated body 4. In the examples shown, the longitudinal axis is vertical. In each magnetizing zone, the magnetic field direction is also aligned perpendicular to a direction of movement of the elongated body 4 while the elongated body 4 is moving into and/or out of (e.g., through) the first processing region 6A. In the examples shown, the direction of movement is horizontal.

The first magnetic field pattern magnetizes the elongated body 4 with a magnetization pattern defined by the first magnetic field pattern in the first processing region 6A. The magnetization pattern is retained by permanent magnetism in the elongated body 4 after the elongated body 4 has left the first processing region 6A.

The material of the elongated body 4 is typically ferromagnetic or ferrimagnetic at room temperature. The material of the elongated body 4 can therefore retain a net magnetization at room temperature without an applied external magnetic field being present. The elongated body 4 may have a range of compositions. The elongated body 4 may, for example, comprise a metallic alloy. In some implementations, the elongated body 4 comprises a ferromagnetic stainless steel, such as a ferritic stainless steel, a martensitic stainless steel, or precipitation hardenable stainless steel. Stainless steel grades 304, 306, 316, martensitic stainless grades, ('400' series) and/or 17-7 may, for example, be used.

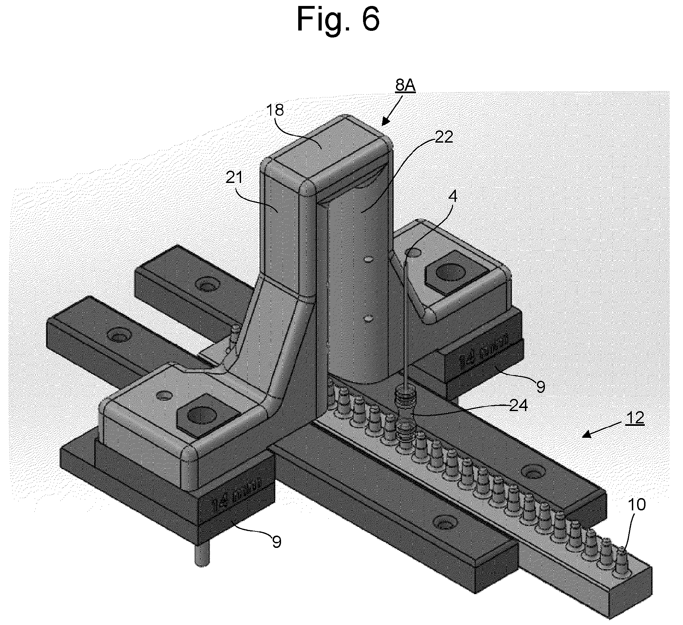

As exemplified in Figures 1 and 6, the apparatus 2 may comprise one or more holders 10 for holding one or more elongated bodies 4. Each holder 10 may hold a single respective elongated body 4 or a respective sub-group of elongated bodies 4. Each holder 10 may comprise a gripping mechanism for gripping an elongated body 4 or a plurality of elongated bodies 4. The gripping mechanism may be actuatable to allow the elongated body or bodies to be selectively held (e.g., held for processing and released to allow for attachment or removal of the elongated bodies 4 from the apparatus 2) or may grip by friction fitting or any other suitable holding approach. In the examples shown, the apparatus comprises a plurality of holders 10 that are configured to hold a corresponding plurality of elongated bodies 4. In the examples shown, each holder 10 comprises a protrusion extending substantially parallel to the longitudinal axis and configured to cooperatively engage within a recess 25 in a hub portion 24 associated with the elongated body 4 to provide a friction fit. The holders 10 hold the elongated bodies 4 in an orientation that is perpendicular to the direction of movement of the elongated bodies 4 through the first processing region 6A of the first magnetizer 8A and ensure a controlled and/or repeatable relative position of the elongated body within the first processing region 6A.

As depicted schematically in Figures 1 and 6, the apparatus 2 may comprise a conveyance arrangement 12. The conveyance arrangement 12 may convey a plurality of the elongated bodies 4 in sequence into and out of (e.g., through) the first processing region 6A of the first magnetizer 8A. The conveyance arrangement 12 allows methods of the disclosure to be applied to a plurality of elongated bodies 4 in sequence. The conveyance arrangement 12 may comprise a conveyor belt or track or the like. The conveyance arrangement 12 may drive movement of holders 10 that hold respective elongated bodies 4.

In the examples shown, the elongated bodies 4 are moved into and out of (e.g., through) the first processing region 6A in single file, one at a time. This may result in the first processing region 6A containing at most only a single elongated body 4 at any given time. The conveyance arrangement 12 may, however, be configured to move more than one elongated body 4 through the first processing region 6A at a time, for example by providing holders 10 in an array having rows perpendicular to the direction of movement of the holders 10 that have two or more holders per row, such as three holders per row, four holders per row, or five holders per row (or are otherwise configured to hold two or more elongated bodies 4 per row). Each row may comprise holders 10 aligned along a line that is perpendicular or oblique to the direction of movement of the elongated bodies 4 into and/or out of (e.g., through) the first processing region 6A.

In some implementations, the hub portion 24 comprises an identifier, such as an RFID tag, that identifies the elongated body 4 associated with the hub portion 24. The identifier may identify a target magnetization pattern that is associated with the elongated body 4 and/or one or more other characteristics of the elongated body 4. When the elongated body 4 is used, for example during a surgical operation, a tracking device may read the identifier to determine a nominal magnetization of the elongated body 4. The tracking device may use the determined nominal magnetization to determine how to track the elongated body 4 optimally.

The first magnetizer 8A may generate the first magnetic field pattern at least partially using one or more permanent magnets 16A-16F. In some implementations, the first magnetizer 8A comprises a plurality of permanent magnets 16A-16F. Example configurations with plural magnets 16A-16F are shown in Figures 2, 4 and 5. In such cases, the first magnetizer 8A generates the first magnetic field pattern using the plurality of magnets 16A-16F. In some implementations, the magnetizer 8A may use other arrangements to generate the first magnetic field pattern. Such other arrangements may be provided in addition to permanent magnets or as an alternative to permanent magnets. For example, one or more electromagnets may be used to generate, or contribute to, the first magnetic field pattern.

As exemplified in Figures 2, 4 and 5, the plurality of magnets 16A-16F may comprise one or more pairs of magnets. The magnets of each pair of magnets may be on opposite sides of the first processing region 6A in a direction perpendicular to the movement of the elongated body 4 into and/or out of (e.g., through) the first processing region 6A. This is illustrated for example in Figure 2, where magnets 16A and 16B are positioned on opposite sides of the first processing region 6A in a horizontal direction within the plane of the page (with the direction of movement of the elongated body 4 being perpendicular to the plane of the page in the orientation shown). Providing magnets in pairs facilitates provision of a strong magnetic field in the first processing region 6A. This is particularly the case where the magnets in each pair of magnets are configured to contribute to the magnetic field strength in the first processing region 6A in the same sense as each other. This may be achieved by arranging the magnets of each pair of magnets to be oriented in the same direction as each other, as exemplified in Figure 2 (with the labels "N" and "S" schematically indicating magnetic poles). The magnets 16A-16B thus both apply a magnetic field to the elongated body 4 that is oriented predominantly vertically downwards in the region between the two magnets 16A-16B. The effect is also promoted by aligning the magnets with each other in a direction perpendicular to the direction of magnetic flux in the magnets (e.g., aligned along the horizontal direction, to be substantially at the same height). The magnets in each pair may be substantially identical to each other to maximise the symmetry of the magnetic field applied to elongated bodies 4 as they pass through the first processing region 6A, for example to increase the extent to which the magnetic field is parallel to the longitudinal axes of the elongated bodies 4 as they pass through the first processing region 6A. The magnets of each pair of magnets may be positioned and oriented to be mirror symmetric in a plane perpendicular to movement of elongated bodies 4 through the first processing region 6A relative to longitudinal axes of the elongated bodies 4.

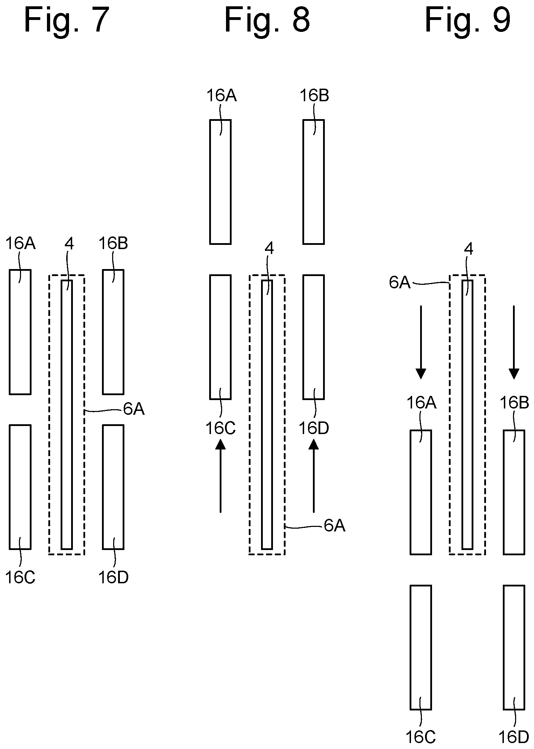

As exemplified in Figure 6, the pairs of magnets 16A-D may be supported by a support structure 18. The support structure 18 may comprise a first column 21 to support one of the magnets of each of the pairs of the magnets. The support structure 18 may comprise a second column 22 to support the other of the magnets of each of the pairs of magnets. Thus, for the pair of magnets shown in Figure 2, for example, the first column 21 could support magnet 16A and the second column 22 could support magnet 16B. The first column 21 and the second column 22 may be connected together to form an arch, as exemplified in Figure 6. Connecting the first and second columns together in this manner provides structural strength as well as facilitating accurate simultaneous movement of the first and second columns, for example where it is desired to simultaneously move all of the magnets 16A-16D, as exemplified in Figures 8 and 9 and discussed in further detail below.

In some implementations, the magnetization pattern retained by the elongated body comprises a plurality of zones having different permanent magnetizations. This may be achieved by the first magnetizer 8A on its own and/or by the first magnetizer 8A in combination with one or more second magnetizers 8B, as discussed further below.

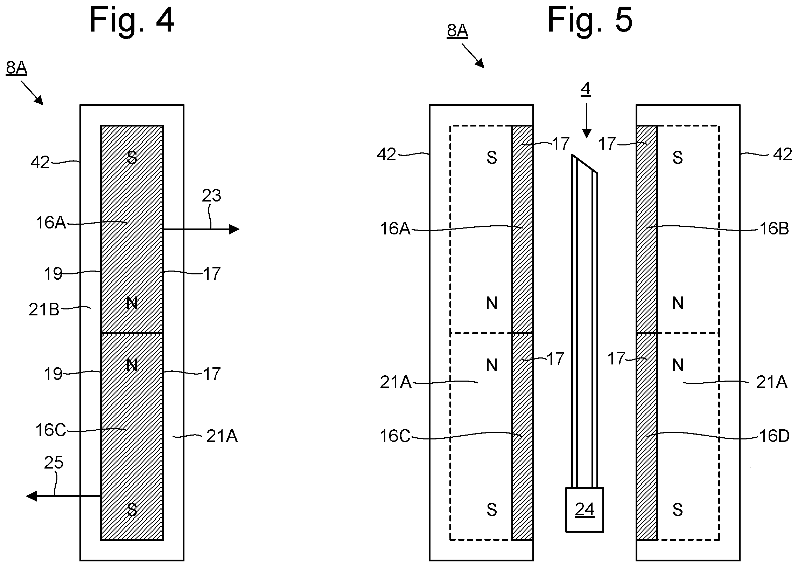

Figures 4 and 5 depict example configurations of a first magnetizer 8A configured to impart a magnetization pattern comprising a plurality of zones having different permanent magnetizations.

The plurality of zones may comprise at least two zones magnetized in a direction parallel to the longitudinal axis (i.e., in the same direction as each other or in opposite directions) and separated from each other by a zone having substantially no permanent magnetization. In arrangements of this type, the elongated body 4 may be preconfigured to have a lower maximum potential residual magnetization (e.g., substantially zero potential residual magnetization) in at least a portion of the zone that has substantially no permanent magnetization than in at least one other portion of the elongated body 4 outside of the zone. This configuration may make it easier to provide a clear distinction between magnetized zones and unmagnetized zones in the magnetization pattern. The lower maximum potential residual magnetization may be defined as the maximum potential residual magnetization that the material is capable of retaining in the absence of an external magnetic field. A lower maximum potential residual magnetization may be achieved in a selected zone of an elongated element 4 by using a different material composition in that zone and/or by processing material in the zone to suppress magnetizability in that zone, for example by localized heat treatment or mechanical working. The suppression of magnetizability may, for example, comprise changing material from being ferromagnetic (or ferrimagnetic) at room temperature to being paramagnetic at room temperature.

Alternatively or additionally, the plurality of zones of retained magnetization may comprise at least two zones in which the permanent magnetization in each zone is opposite in direction to the other zone and parallel to the longitudinal axis of the elongated body 4. Figures 4 and 5 depict an example configuration for achieving such a plurality of zones. In the example of Figure 4, two pairs of magnets 16A-B and 16C-D are configured to provide two zones of different magnetization. A first region between magnets 16A-B provides a first zone in which magnetization is oriented vertically upwards. A second region between magnets 16C-D provide a second zone in which magnetization is orientated vertically downwards.

As exemplified in Figures 4 and 5, in some configurations the plurality of magnets 16A-16D are supported by a mounting arrangement containing one or more field-guiding bodies 42. The field-guiding bodies 42 may be provided in a support structure 18 of the mounting arrangement. Each field-guiding body 42 is formed from a material having a high relative magnetic permeability, such as greater than one, optionally greater than 500, optionally greater than 5000, optionally greater than 10000. The field-guiding bodies 42 may comprise any material having a suitably high relative magnetic permeability, including for example one or more of the following: iron, mu-metal, cobalt-iron, a steel, a stainless, and a ferrite. The field-guiding bodies 42 are shaped to promote efficient magnetization of the elongated body 4 by concentrating magnetic fields associated with the magnets 16A-16D in regions of interest. For example, the one or more field-guiding bodies 42 may be configured to inhibit spreading of a magnetic field from the one or more permanent magnets 16A-16D outside of the magnetizing zone. Inhibiting such spreading increases the strength of the magnetic field in the magnetizing zone as well as reducing or avoiding application of a magnetic field having significant components oriented perpendicularly to the longitudinal axis of the elongated body while the elongated body is approaching the magnetizing zone or moving away from the magnetizing zone. The quality of the magnetization applied to the elongated body may thereby be improved.

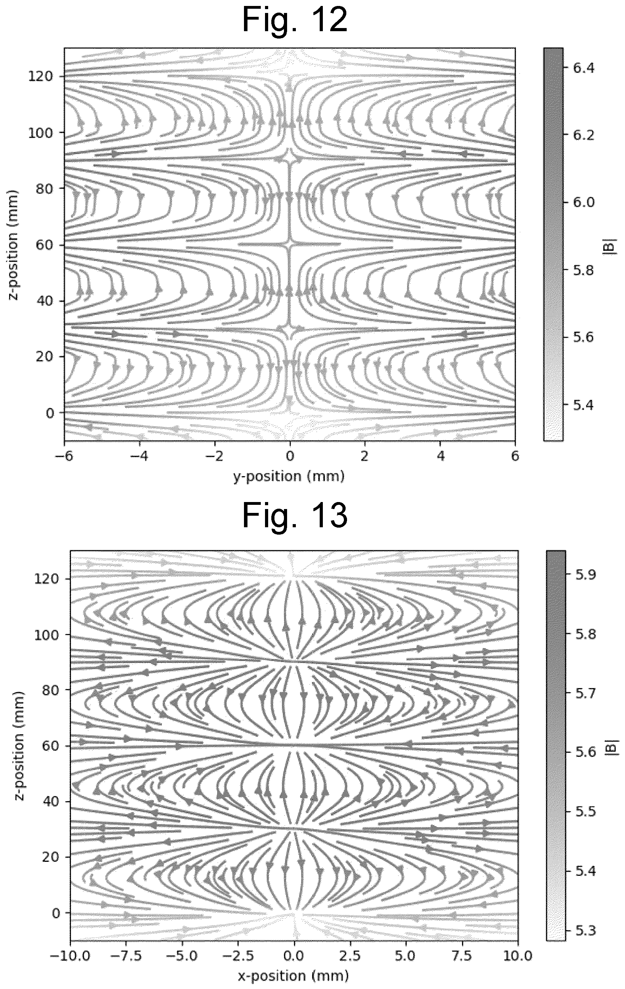

The simulated magnetic fields shown in Figures 12 and 13 illustrate the benefits of avoiding such spreading. The magnetic fields shown Figures 12 and 13 are generated from two columns of magnets stacked in the manner shown in Figures 4 and 5 except with four magnets in each column rather than two and without any field-guiding bodies. Figure 12 shows the magnetic field distribution in the Y-Z plane looking along the direction of movement of the elongated body 4 with the columns of magnets positioned at *y* = -6*mm* and *y* = +6*mm.* It can be seen that the magnetic field is orientated along the Z axis at *y* = 0 and will therefore be aligned as desired with the longitudinal axis of the elongated body 4 when the elongated body 4 is in the Y-Z plane (i.e., in the magnetizing zone of the first processing region). Figure 13, on the other hand, shows how the magnetic field varies in the X-Z plane. During processing, the elongated body 4 will move along the X-axis from left to right in the X-Z plane. When the elongated body 4 passes through x = 0 (i.e., passes through the Y-Z plane and magnetizing zone), the magnetic field is predominantly aligned along the longitudinal axis of the elongated body 4 as mentioned above in relation to Figure 12. However, when the elongated body 4 is away from *x* = 0, for example as the elongated body approaches *x* = 0 from the left or moves away from *x* = 0 to the right, it can be seen that the magnetic field is no longer aligned vertically and instead has significant horizontal components that vary along the Z direction (along the height). These deviations can disrupt magnetization of the elongated body 4 with a desired magnetization pattern. The provision of field guiding bodies 42 to inhibit spreading of the magnetic field away from the magnetizing zone (e.g., spreading along the positive and negative X directions away from *x* = 0) reduces or completely suppresses the magnetic field strength in the regions where the magnetic field would not be oriented correctly (e.g., not oriented along the longitudinal axis of the elongated body 4), thereby reducing unwanted disruption of the magnetization of the elongated body 4.

In the particular example of Figures 4 and 5, two field-guiding bodies 42 are provided. Each field-guiding body 42 is shaped in the form of a trough. An opening of each trough faces towards the magnetizing zone. The openings of the two troughs face each other across the magnetizing zone. In the example shown, two bar magnets are provided in each field-guiding body 42 to form two pairs of bar magnets facing each other across the magnetizing zone.

In one class of implementation, as exemplified in Figures 4 and 5, each of the permanent magnets 16A-16D has a forward face 17 and a rear face 19. The forward face 17 is positioned to face in a forward direction 23 that is predominantly along a direction of movement of the elongated body 4 out of the first processing region (e.g., along a positive X direction, to the right in Figure 4). The rear face 19 is positioned to face in a rearward direction 25 that is predominantly opposite to the direction of movement of the elongated body 4 out of the first processing region (e.g., along a negative X direction, to the left in Figure 4). The one or more field-guiding bodies 42 comprises a forward-shielding field-guiding portion 21A. In the example of Figures 4 and 5, each of the two field-guiding bodies 42 comprises a separate forward-shielding field-guiding portion 21A. The forward-shielding field-guiding portion 21A is positioned outside of the forward face 17 along the forward direction (e.g., positive X direction). The forward-shielding field-guiding portion 21A thus effectively shields a region in front of the forward-shielding field-guiding portion 21A from magnetic fields generated by the permanent magnets on the other side of the forward-shielding field-guiding portion 21A, thereby inhibiting spreading of the magnetic field in the forwards direction. Alternatively or additionally, a rearward-shielding field-guiding portion 21B may be provided. The rearward-shielding field-guiding portion 21B is positioned outside of the rear face 19 along the rearward direction (e.g., negative X direction). The rearward-shielding field-guiding portion 21B thus effectively shields a region behind the rearward-shielding field-guiding portion 21B from magnetic fields generated by the permanent magnets on the other side of the rearward-shielding field-guiding portion 21B, thereby inhibiting spreading of the magnetic field in the rearward direction. The forward-shielding field-guiding portion 21A may typically be more important than the rear-shielding field-guiding portion 21B because magnetization errors introduced before the elongated body 4 has entered the first processing region may be corrected by the magnetization applied in the first processing region whereas magnetization errors introduced after the elongated body 4 has left the first processing region may not be so corrected.

As mentioned above, in some implementations the first magnetizer generates the first magnetic field at least partially using an electromagnet. Using an electromagnet provides increased flexibility for controlling the magnetic field in the first processing region, allowing for example for the magnetic field to be turned on and off quickly without requiring mechanical movement of any elements. In such implementations, it may be possible to address the challenges discussed above with reference to Figures 12 and 13 by controlling activation of the electromagnet as a function of position of the elongated body 4 as it is moved relative to the first processing region. For example, the electromagnet may be controlled to selectively generate the first magnetic field pattern when the elongated body 4 is in the first processing region and not generate the first magnetic field pattern when the elongated body 4 is outside of the first processing region. Such selective control may, for example comprise deactivating the electromagnet when the elongated body 4 is approaching the first processing region before moving into the first processing region and/or when the elongated body 4 is moving away from the first processing region after moving out of the first processing region. This may allow high quality magnetization of the elongate body 4 to be achieved even for magnetic field distributions that spread significantly outside of the first processing region, such as that depicted in Figure 13. This may obviate the need for field-guiding bodies or act to further improve the quality and reliability of magnetization of the elongated body 4 achieved by the method.

In some implementations, as exemplified in Figure 1, the method further comprises moving the elongated body 4 into and/or out of (e.g., through) one or more second processing regions 6B of respective one or more second magnetizers 8B. In the example of Figure 1, a single second magnetizer 8B is provided, but it will be appreciated that more than one second magnetizer 8B may be provided, such as two, three, four, or five second magnetizers. Each second magnetizer 8B is configured to generate a second magnetic field pattern in the respective second processing region 6B. The second magnetic field pattern is different from the first magnetic field pattern generated by the first magnetizer 8A. Where a plurality of second magnetizers 8B are provided, the corresponding plurality of second magnetic field patterns may all be different from each other and different from the first magnetic field pattern.

The second magnetic field pattern may take any of the forms described above for the first magnetic field pattern.

In some implementations, one or more of the second magnetizers 8B may have the same structure as the first magnetizer 8B but be configured such that the relative positioning of one or more magnets of the second magnetizer 8B (e.g., raised, lowered or moved laterally, as discussed in further detail below with reference to Figures 7-11) relative to the second processing region 8B is different from the relative positioning of corresponding one or more magnets of the first magnetizer 8A relative to the first processing region 8A. Referring to Figures 7-8 for example, the first magnetizer 8A may be configured as shown in Figure 7 while a second magnetizer 8B is configured as shown in Figure 8 or 9.

Passing the elongated body 4 through multiple different magnetic field patterns facilitates provision of complex patterns of magnetization in the elongated body 4, including avoiding or reducing the need for individual magnetizers to have a complex structure, reducing or avoiding the need for individual permanent magnets to be excessively small, and/or facilitating the provision of magnetic field strengths in the processing region that are sufficiently large to ensure reliable magnetization of the elongated body 4.

In some implementations, as exemplified in Figure 1, the apparatus 2 further comprises a sensing module 30 configured to measure at least an aspect of magnetization patterns retained in elongated bodies after they have been processed, for example after each elongated body has left the first processing region 6A of a first magnetizer 8A and, where present, the second processing region 6B of each of one or more second magnetizers 8B. The sensing module 30 may comprise any suitable combination of hardware, firmware and software for performing the measurements. The sensing module 30 may comprise one or more magnetic field sensors 34 configured to measure a magnetic field, such as one or more Hall-effect sensors. Where a plurality of magnetic field sensors 34 are provided, as exemplified in Figure 1, the sensors 34 may be provided at different locations to obtain more detailed information about the retained magnetism in the elongated body 4 being measured. The measurement of the retained magnetization pattern may be performed using the one or more magnetic field sensors 34 while the elongate body 4 is held at a predetermined location and orientation relative to the one or more magnetic field sensors 34 during the measurement of the retained magnetization pattern. Holding the elongate body 4 at a predetermined location and orientation facilitates comparison of measurements of the one or more magnetic field sensors 34 with expected measurements for a particular magnetization pattern. This supports more accurate assessment of the magnetization pattern. An apparatus may thus be provided for assessing a magnetization pattern in an elongate body. The apparatus may comprise one or more magnetic field sensors. The apparatus may comprise a holder configured to hold the elongate body 4 at a predetermined location and orientation relative to the one or more magnetic field sensors 34. In one implementation, the one or more magnetic field sensors are provided in an ultrasound head and the holder is configured to hold the elongate body 4 (e.g., via one or more holes or slots) at a predetermined location and orientation relative to the ultrasound head. A data processing system may be provided that is configured to: cause the one or more magnetic field sensors 34 to measure a magnetic characteristic of the elongate body 4; compare the measured magnetic characteristic with an expected magnetic characteristic associated with a target magnetization pattern; and determine a quality of a magnetization pattern of the elongate body 4 based on the comparison.

In the example of Figure 1, the sensing module 30 is provided downstream of a first magnetizer 8A and a single second magnetizer 8B. The apparatus 2 is further configured to use the sensing module 30 to evaluate a quality of the magnetization pattern retained in the elongated body 4. This may, for example, involve processing measurement data obtained by the sensing module 30 to compare the sensed magnetization pattern with a target magnetization pattern. A degree of matching may be determined between the sensed magnetization pattern and the target magnetization pattern. When the degree of matching is determined to be sufficiently good, for example when a quality metric is higher than a predetermined threshold, the apparatus 2 may provide an output to indicate that the processed elongated body 4 is correctly magnetized. The output may take any suitable form, such as a data stream for use remotely, for example at a remote monitoring computer or mobile device, and/or an output for providing immediate local information to a user, such as an output on a display or an output via an LED (e.g., a green light) etc. The processing of the measurement data may be performed in the sensing module 30 or in a device separate from the sensing module 30. In the example of Figure 1, the processing is performed by a control module 32, which is discussed further below. When the degree of matching is determined to be unsatisfactory, for example when the quality metric is lower than the predetermined threshold, the apparatus 2 may provide an output to indicate that the processed elongated body 4 has not be correctly magnetized. The output may take any of the forms described above. In the case where the output includes output via an LED, the light may be red instead of green for example. When an elongated body 4 is identified as having been incorrectly magnetized the elongated body 4 may be rejected or put in a queue to be processed again by the apparatus 2.

In some implementations, as exemplified in Figure 1, the sensing module 30 may additionally or alternatively comprise one or more position sensors 36 for detecting a position of the elongated body 4. For example, the position sensor 36 may measure the position of a tip of the elongated body 4 to determine whether the elongated body 4 is correctly mounted on the conveyance arrangement 12. Incorrect positioning of an elongated body 4 while the elongated body 4 is being moved through the processing regions 6A and/or 6B of the first and/or second magnetizers 8A, 8B, for example such that a tip of the elongated body 4 is higher or lower than an expected position for the tip, may result in unwanted deviations in the magnetization pattern imparted to the elongated body 4 relative to a target magnetization pattern. The apparatus 2 may be configured to provide an output in response to detection of incorrect and/or correct positioning. The output or outputs may take any of the forms discussed above in the context of responding to measurements of retained magnetization patterns.

As exemplified in Figure 1, the apparatus 2 may comprise a control module 32 for controlling operation of the apparatus 2. The control module 32 may comprise any suitable combination of data processing hardware, firmware and/or software necessary to perform the processing required. As mentioned above, the control module 32 may interact with a sensing module 30 to monitor the quality of the magnetization process and/or positioning of elongated bodies. Alternatively or additionally, the control module 32 may control operation of one or more other aspects of the apparatus 2, such as the first magnetizer 8A, any second magnetizer 8B, and/or the conveyance arrangement 12. For example, where the first and/or second magnetizer 8A, 8B is configured to drive movement of one or more magnets 16A-16D, the control module 32 may interact with hardware that is configured to provide the movements, including any suitable combination of moveable mounting arrangements, motors, sensors, etc., to provide the required movements (e.g., to position the permanent magnets where they are required at the appropriate times). The control module 32 may respond to feedback from the sensing module 30 to adjust and/or optimise adjustable parameters of the method, such as the positioning of magnets and/or elongated bodies and/or the speed at which elongated bodies are conveyed through the processing regions.

In examples where the first magnetizer 8A comprises a plurality of permanent magnets 16A-16F, the plurality of magnets may be supported by a mounting arrangement. The mounting arrangement may be configured to allow the magnets 16A-16F to be moved relative to the first processing region 6A.

Referring to Figures 2 and 6, the mounting arrangement may comprise a support structure 18. The magnets 16A-16F are supported by the support structure 18. The magnets 16A-16F may be rigidly attached to the support structure 18. The movement of the magnets 16A-16F may be achieved in this case by moving the support structure 18. Motors and/or sensors may be provided for driving and sensing movement of the support structure 18. Alternatively or additionally, the mounting arrangement may be configured to allow a user to manually change a position of the support structure 18 in the mounting arrangement, for example by manually disconnecting the support structure 18 from a first position in the mounting arrangement and reconnecting the support structure at a second position in the mounting arrangement.

In some implementations, the support structure 18 is configured to allow the magnets to move relative to the support structure 18, for example by mounting the magnets on tracks and/or arms. Motors and/or sensors may be provided for driving and sensing movement of the magnets along the tracks and/or for driving movement of the arms.

The mounting arrangement may be configured to allow movement of one or more of the magnets in a direction parallel to the longitudinal axis. Figure 6 is a schematic perspective view of an example apparatus 2 having a mounting arrangement of this type. In the example shown, the support structure 18 is attached to a moveable platform 9. Vertical movement of the platform 9 causes a corresponding vertical movement of the support structure 18. Vertical positions of magnets rigidly mounted to the support structure 18 can thus be changed by moving the support structure 18 along the vertical axis. Figures 7-9 depict three different positions of permanent magnets 16A-16D relative to an elongated body 4 achievable by vertical movement of a support structure 18 supporting the magnets 16A-16D.

Figure 7 depicts an example configuration in which a centre of mass of the group of magnets 16A-16D is substantially aligned in height with a centre of mass of the elongated body 4, similar to the situation depicted in Figures 1 and 2. This configuration allows a relatively large proportion of the elongated body 4 to be magnetized by the permanent magnets.

Figure 8 depicts an example configuration in which the magnets 16A-16D have been moved upwards relative to the case in Figure 7, such that the lowermost magnets 16C-16D are laterally aligned with an upper portion of the elongated body 4 and the uppermost magnets 16A-16B are above an uppermost tip of the elongated body 4. This configuration allows an upper portion of the elongated body 4 to be magnetized while a lower portion of the elongated body 4 is substantially unaffected by the magnets 16A-16D. In comparison with the arrangement of Figure 7, the vertical movement of the magnets 16A-16D effectively switches off a lower portion of the first processing region 6A, such that only the upper portion of the elongated body 4 is processed (e.g., magnetized).

Figure 9 depicts an example configuration in which the permanent magnets 16A-16D have been moved downwards relative to the case in Figure 7, such that the uppermost magnets 16A-16B are laterally aligned with a lower portion of the elongated body 4 and the lowermost magnets 16C-D are below a lowermost extremity of the elongated body 4. This configuration allows a lower portion of the elongated body 4 to be magnetized while an upper portion of the elongated body 4 is substantially unaffected by the magnets 16A-16D. In comparison with the arrangement of Figure 7, the vertical movement of the magnets 16A-16D effectively switches off an upper portion of the first processing region 6A, such that only the lower portion of the elongated body 4 is processed (e.g., magnetized).

In some implementations, the mounting arrangement is configured to allow movement of one or more of the magnets 16A-16D in a direction perpendicular to the longitudinal axis. This may be implemented, for example, by mounting the magnets 16A-D on actuatable rails or arms. The direction perpendicular to the longitudinal axis will typically be a direction perpendicular or oblique to the direction of movement of the elongated body 4 through the first processing region 6A.

Figures 10-11 depict two example sets of positions of magnets 16A-16D relative to an elongated body 4 achievable by horizontal movement of the magnets 16A-16D.

Figure 10 depicts an example configuration in which the lowermost magnets 16C-D have been moved horizontally outwards relative to the configuration of Figure 7. This configuration allows an upper portion of the elongated body 4 to be magnetized by the uppermost permanent magnets 16A-B while a lower portion of the elongated body 4 is substantially unaffected by the magnets 16C-16D. In comparison with the arrangement of Figure 7, the horizontal movement of the magnets 16C-16D effectively switches off a lower portion of the first processing region 6A, such that only the upper portion of the elongated body 4 is processed (e.g., magnetized). The effect achieved is thus similar to that of the configuration of Figure 8 discussed above.

Figure 11 depicts an example configuration in which the uppermost magnets 16A-16B have been moved horizontally outwards relative to the configuration of Figure 7. This configuration allows a lower portion of the elongated body 4 to be magnetized by the lowermost permanent magnets 16C-16D while an upper portion of the elongated body 4 is substantially unaffected by the magnets 16A-16D. In comparison with the arrangement of Figure 7, the horizontal movement of the magnets 16A-16B effectively switches off an upper portion of the first processing region 6A, such that only the lower portion of the elongated body 4 is processed (e.g., magnetized). The effect achieved is thus similar to that of the configuration of Figure 9 discussed above.

The following numbered clauses define embodiments of the disclosure.
1. A method of processing an elongated body, comprising:
   moving an elongated body into and out of a first processing region associated with a first magnetizer, wherein:
   the first magnetizer generates a first magnetic field pattern in the first processing region, the first magnetic field pattern comprising a magnetizing zone in which a magnetic field direction is predominantly aligned with a longitudinal axis of the elongated body and is perpendicular to a direction of movement of the elongated body while the elongated body is moving into the first processing region; and
   the first magnetic field pattern magnetizes the elongated body with a magnetization pattern defined by the first magnetic field pattern in the first processing region, the magnetization pattern being retained by permanent magnetism in the elongated body after the elongated body has left the first processing region.
2. The method of clause 1, wherein the first magnetizer generates the first magnetic field pattern at least partially using one or more permanent magnets.
3. The method of clause 2, wherein the one or more magnets are supported by a mounting arrangement configured to allow the one or more magnets to be moved relative to the first processing region.
4. The method of clause 3, wherein the mounting arrangement is configured to allow movement of one or more of the magnets in a direction parallel to the longitudinal axis.
5. The method of clause 3 or 4, wherein the mounting arrangement is configured to allow movement of one or more of the magnets in a direction perpendicular to the longitudinal axis.
6. The method of any of clauses 2-5, wherein the one or more magnets comprises one or more pairs of magnets.
7. The method of clause 6, wherein the magnets of each pair of magnets are positioned on opposite sides of the first processing region in a direction perpendicular to the movement of the elongated body into and out of the first processing region.
8. The method of clause 6 or 7, wherein the magnets of each pair of magnets are substantially identical.
9. The method of any of clauses 6-8, wherein the magnets of each pair of magnets are positioned and oriented to be mirror symmetric in a plane perpendicular to movement of the elongated body into and out of the first processing region, relative to the longitudinal axis of the elongated body.
10. The method of any of clauses 6-9, wherein the one or more pairs of magnets are supported by a support structure comprising:
   a first column to support one of the magnets of each of the pairs of magnets; and
   a second column to support the other of the magnets of each of the pairs of magnets.
11. The method of any of clauses 2-10, wherein the plurality of magnets are supported by a mounting arrangement containing one or more field-guiding bodies, each field-guiding body being formed from a material having a relative magnetic permeability greater than one, optionally greater than 500, optionally greater than 5000, optionally greater than 10000.
12. The method of clause 11, wherein the one or more field-guiding bodies are configured to inhibit spreading of a magnetic field from the one or more permanent magnets outside of the magnetizing zone.
13. The method of clause 11 or 12, wherein:
   each of the permanent magnets has a forward face and a rear face;
   the forward face is positioned to face in a forward direction that is predominantly along a direction of movement of the elongated body out of the first processing region;
   the rear face is positioned to face in a rearward direction that is predominantly opposite to the direction of movement of the elongated body out of the first processing region; and
   the one or more field-guiding bodies comprises a forward-shielding field-guiding portion positioned outside of the forward face along the forward direction and/or a rearward-shielding field-guiding portion outside of the rear face along the rearward direction.
14. The method of any preceding clause, wherein the first magnetizer generates the first magnetic field at least partially using an electromagnet.
15. The method of clause 14, comprising controlling the electromagnet to:
   generate the first magnetic field pattern when the elongated body is in the first processing region; and
   not generate the first magnetic field pattern when the elongated body is outside of the first processing region, optionally including when the elongated body is approaching the first processing region before moving into the first processing region and/or when the elongated body is moving away from the first processing region after moving out of the first processing region.
16. The method of any preceding clause, wherein the elongated body is moved by a conveyance arrangement configured to convey a plurality of the elongated bodies in sequence into and out of the first processing region of the first magnetizer.
17. The method of any preceding clause, wherein the method is applied to each of a plurality of the elongated bodies in sequence.
18. The method of any preceding clause, wherein the magnetization pattern comprises a plurality of zones having different permanent magnetizations, optionally wherein the plurality of zones comprises at least two zones in which the permanent magnetization is opposite in direction and parallel to the longitudinal axis of the elongated body.
19. The method of clause 18, wherein the plurality of zones comprises at least two zones magnetized in a direction parallel to the longitudinal axis and separated from each other by a zone having substantially no permanent magnetization.
20. The method of clause 19, wherein the elongated body is preconfigured to have a lower maximum potential residual magnetization, optionally substantially zero maximum potential residual magnetization, in at least a portion of the zone having substantially no permanent magnetization than in at least one other portion of the elongated body outside of the zone.
21. The method of any preceding clause, comprising moving the elongated body into and out of one or more second processing regions associated with respective one or more second magnetizers, each second magnetizer being configured to generate a second magnetic field pattern in the second processing region that is different from the first magnetic field pattern in the first processing region.
22. The method of any preceding clause, further comprising measuring at least an aspect of the retained magnetization pattern after the elongated body has left the first processing region and evaluating a quality of the retained magnetization pattern based on the measurement.
23. The method of clause 22, wherein the measurement of the retained magnetization pattern is performed using one or more magnetic field sensors and the elongate body is held at a predetermined location and orientation relative to the one or more magnetic field sensors during the measurement of the retained magnetization pattern.
24. The method of any preceding clause, wherein the elongated body is an elongated body for a surgical procedure, optionally comprising a portion or all of one or more of the following: a needle, a cannula, a stylet, a guidewire, and a cable.
25. A method of manufacturing a medical instrument, comprising:
   providing an elongated body; and
   processing the elongated body using the method of any preceding clause.
26. A method of assessing a magnetization pattern in an elongate body, comprising:
   holding the elongate body at a predetermined location and orientation relative to one or more magnetic field sensors;
   using the one or more magnetic field sensors to measure a magnetic characteristic of the elongate body while the elongate body is held at the predetermined location and orientation;
   comparing the measured magnetic characteristic with an expected magnetic characteristic associated with a target magnetization pattern; and
   determining a quality of a magnetization pattern of the elongate body based on the comparison.
27. An apparatus for processing an elongated body, comprising:
   a first magnetizer configured to generate a first magnetic field pattern in a first processing region; and
   a conveyance arrangement configured to move an elongated body into and out of the first processing region, wherein:
      the first magnetic field pattern comprises a magnetizing zone in which a magnetic field direction is aligned with a longitudinal axis of the elongated body and is perpendicular to a direction of movement of the elongated body while the elongated body is moving into the first processing region; and
      the first magnetic field pattern is configured to magnetize the elongated body with a magnetization pattern defined by the first magnetic field pattern in the first processing region, the magnetization pattern being retained by permanent magnetism in the elongated body after the elongated body has left the first processing region.
28. The apparatus of clause 27, wherein the first magnetizer comprises one or more permanent magnets and is configured to generate the first magnetic field pattern at least partially using the one or more permanent magnets.
29. The apparatus of clause 28, comprising a mounting arrangement configured to support the one or more magnets, the mounting arrangement configured to allow the one or more magnets to be moved relative to the first processing region.
30. The apparatus of clause 29, wherein the mounting arrangement is configured to allow movement of one or more of the magnets in a direction parallel to the longitudinal axis.
31. The apparatus of clause 29 or 30, wherein the mounting arrangement is configured to allow movement of one or more of the magnets in a direction perpendicular to the longitudinal axis.
32. The apparatus of any of clauses 28-31, wherein the one or more magnets comprises one or more pairs of magnets.
33. The apparatus of clause 32, wherein the magnets of each pair of magnets are positioned on opposite sides of the first processing region in a direction perpendicular to the movement of the elongated body into and out of the first processing region.
34. The apparatus of clause 32 or 33, wherein the magnets of each pair of magnets are substantially identical.
35. The apparatus of any of clauses 32-34, wherein the magnets of each pair of magnets are positioned and oriented to be mirror symmetric in a plane perpendicular to movement of the elongated body into and out of the first processing region, relative to the longitudinal axis of the elongated body.
36. The apparatus of any of clauses 32-35, wherein the one or more pairs of magnets are supported by a support structure comprising:
   a first column to support one of the magnets of each of the pairs of magnets; and
   a second column to support the other of the magnets of each of the pairs of magnets.
37. The apparatus of any of clauses 28-36, wherein the plurality of magnets are supported by a mounting arrangement containing one or more field-guiding bodies, each field-guiding body being formed from a material having a relative magnetic permeability greater than one, optionally greater than 500, optionally greater than 5000, optionally greater than 10000.
38. The apparatus of clause 37, wherein the one or more field-guiding bodies are configured to inhibit spreading of a magnetic field from the one or more permanent magnets outside of the magnetizing zone.
39. The apparatus of clause 37 or 38, wherein:
   each of the permanent magnets has a forward face and a rear face;
   the forward face is positioned to face in a forward direction that is predominantly along a direction of movement of the elongated body out of the first processing region;
   the rear face is positioned to face in a rearward direction that is predominantly opposite to the direction of movement of the elongated body out of the first processing region; and
   the one or more field-guiding bodies comprises a forward-shielding field-guiding portion positioned outside of the forward face along the forward direction and/or a rearward-shielding field-guiding portion outside of the rear face along the rearward direction.
40. The apparatus of any of clauses 27-39, wherein the first magnetizer comprises an electromagnet and is configured to generate the first magnetic field at least partially using the electromagnet.
41. The apparatus of clause 40, comprising a control module configured to control the electromagnet to:
   generate the first magnetic field pattern when the elongated body is in the first processing region; and
   not generate the first magnetic field pattern when the elongated body is outside of the first processing region, optionally including when the elongated body is approaching the first processing region before moving into the first processing region and/or when the elongated body is moving away from the first processing region after moving out of the first processing region.
42. The apparatus of any of clauses 27-41, wherein the conveyance arrangement is configured to convey a plurality of the elongated bodies in sequence into and out of the first processing region of the first magnetizer.
43. The apparatus of any of clauses 27-42, wherein the apparatus is configured to process each of a plurality of the elongated bodies in sequence.
44. The apparatus of any of clauses 27-43, wherein the magnetization pattern comprises a plurality of zones having different permanent magnetizations.
45. The apparatus of clause 44, wherein the plurality of zones comprises at least two zones in which the permanent magnetization is opposite in direction and parallel to the longitudinal axis of the elongated body.
46. The apparatus of clause 44 or 45, wherein the plurality of zones comprises at least two zones magnetized in a direction parallel to the longitudinal axis and separated from each other by a zone having substantially no permanent magnetization.
47. The apparatus of any of clauses 27-46, comprising moving the elongated body into and out of one or more second processing regions associated with respective one or more second magnetizers, each second magnetizer being configured to generate a second magnetic field pattern in the second processing region that is different from the first magnetic field pattern in the first processing region.
48. The apparatus of any of clauses 27-47, further comprising:
   one or more magnetic field sensors configured to measure at least an aspect of the retained magnetization pattern after the elongated body has left the first processing region; and
   a data processing system configured to evaluate a quality of the retained magnetization pattern based on the measurement.
49. The apparatus of clause 48, further comprising a holder configured to hold the elongate body at a predetermined location and orientation relative to the one or more magnetic field sensors during the measurement of the retained magnetization pattern.
50. The apparatus of any of clauses 27-49, wherein the elongated body is an elongated body for a surgical procedure, optionally comprising a portion or all of one or more of the following: a needle, a cannula, a stylet, a guidewire, and a cable.
51. An apparatus for assessing a magnetization pattern in an elongate body, comprising:
   one or more magnetic field sensors;
   a holder configured to hold an elongate body at a predetermined location and orientation relative to the one or more magnetic field sensors; and
   a data processing system configured to:
      cause the one or more magnetic field sensors to measure a magnetic characteristic of the elongate body;
      compare the measured magnetic characteristic with an expected magnetic characteristic associated with a target magnetization pattern; and
   determine a quality of a magnetization pattern of the elongate body based on the comparison.

## Claims

1. A method of processing an elongated body, comprising:
moving an elongated body into and out of a first processing region associated with a first magnetizer, wherein:
the first magnetizer generates a first magnetic field pattern in the first processing region, the first magnetic field pattern comprising a magnetizing zone in which a magnetic field direction is predominantly aligned with a longitudinal axis of the elongated body and is perpendicular to a direction of movement of the elongated body while the elongated body is moving into the first processing region; and
the first magnetic field pattern magnetizes the elongated body with a magnetization pattern defined by the first magnetic field pattern in the first processing region, the magnetization pattern being retained by permanent magnetism in the elongated body after the elongated body has left the first processing region.

2. The method of claim 1, wherein the first magnetizer generates the first magnetic field pattern at least partially using one or more permanent magnets.

3. The method of claim 2, wherein the one or more magnets are supported by a mounting arrangement configured to allow the one or more magnets to be moved relative to the first processing region, optionally in a direction parallel to the longitudinal axis.

4. The method of claim 2 or 3, wherein the one or more magnets comprises one or more pairs of magnets, wherein the magnets of each pair of magnets are positioned on opposite sides of the first processing region in a direction perpendicular to the movement of the elongated body into and out of the first processing region,
optionally wherein:
the magnets of each pair of magnets are positioned and oriented to be mirror symmetric in a plane perpendicular to movement of the elongated body into and out of the first processing region, relative to the longitudinal axis of the elongated body; and/or
the one or more pairs of magnets are supported by a support structure comprising:
a first column to support one of the magnets of each of the pairs of magnets; and
a second column to support the other of the magnets of each of the pairs of magnets.

5. The method of any of claims 2-4, wherein the plurality of magnets are supported by a mounting arrangement containing one or more field-guiding bodies, each field-guiding body being formed from a material having a relative magnetic permeability greater than one, optionally greater than 500, optionally greater than 5000, optionally greater than 10000, wherein the one or more field-guiding bodies are optionally configured to inhibit spreading of a magnetic field from the one or more permanent magnets outside of the magnetizing zone.

6. The method of claim 5, wherein:
each of the permanent magnets has a forward face and a rear face;
the forward face is positioned to face in a forward direction that is predominantly along a direction of movement of the elongated body out of the first processing region;
the rear face is positioned to face in a rearward direction that is predominantly opposite to the direction of movement of the elongated body out of the first processing region; and
the one or more field-guiding bodies comprises a forward-shielding field-guiding portion positioned outside of the forward face along the forward direction and/or a rearward-shielding field-guiding portion outside of the rear face along the rearward direction.

7. The method of any preceding claim, wherein:
the first magnetizer generates the first magnetic field at least partially using an electromagnet; and
the method comprises controlling the electromagnet to:
generate the first magnetic field pattern when the elongated body is in the first processing region; and
not generate the first magnetic field pattern when the elongated body is outside of the first processing region, optionally including when the elongated body is approaching the first processing region before moving into the first processing region and/or when the elongated body is moving away from the first processing region after moving out of the first processing region.

8. The method of any preceding claim, wherein:
the elongated body is moved by a conveyance arrangement configured to convey a plurality of the elongated bodies in sequence into and out of the first processing region of the first magnetizer; and/or
the method is applied to each of a plurality of the elongated bodies in sequence; and/or
the elongated body is an elongated body for a surgical procedure, optionally comprising a portion or all of one or more of the following: a needle, a cannula, a stylet, a guidewire, and a cable.

9. The method of any preceding claim, wherein the magnetization pattern comprises a plurality of zones having different permanent magnetizations, optionally wherein the plurality of zones comprises at least two zones in which the permanent magnetization is opposite in direction and parallel to the longitudinal axis of the elongated body.

10. The method of claim 9, wherein the plurality of zones comprises at least two zones magnetized in a direction parallel to the longitudinal axis and separated from each other by a zone having substantially no permanent magnetization.

11. The method of claim 10, wherein the elongated body is preconfigured to have a lower maximum potential residual magnetization in at least a portion of the zone having substantially no permanent magnetization than in at least one other portion of the elongated body outside of the zone.

12. The method of any preceding claim, comprising moving the elongated body into and out of one or more second processing regions associated with respective one or more second magnetizers, each second magnetizer being configured to generate a second magnetic field pattern in the second processing region that is different from the first magnetic field pattern in the first processing region.

13. The method of any preceding claim, further comprising measuring at least an aspect of the retained magnetization pattern after the elongated body has left the first processing region and evaluating a quality of the retained magnetization pattern based on the measurement, optionally wherein the measurement of the retained magnetization pattern is performed using one or more magnetic field sensors and the elongate body is held at a predetermined location and orientation relative to the one or more magnetic field sensors during the measurement of the retained magnetization pattern.

14. A method of manufacturing a medical instrument, comprising:
providing an elongated body; and
processing the elongated body using the method of any preceding claim.

15. An apparatus for processing an elongated body, comprising:
a first magnetizer configured to generate a first magnetic field pattern in a first processing region; and
a conveyance arrangement configured to move an elongated body into and out of the first processing region, wherein:
the first magnetic field pattern comprises a magnetizing zone in which a magnetic field direction is aligned with a longitudinal axis of the elongated body and is perpendicular to a direction of movement of the elongated body while the elongated body is moving into the first processing region; and
the first magnetic field pattern is configured to magnetize the elongated body with a magnetization pattern defined by the first magnetic field pattern in the first processing region, the magnetization pattern being retained by permanent magnetism in the elongated body after the elongated body has left the first processing region.
